(19) Europäisches Patentamt
European Patent Office
Office européen des brevets

(11) EP 0 590 709 B1

(12) FASCICULE DE BREVET EUROPEEN

(45) Date de publication et mention
de la délivrance du brevet:
**29.07.1998 Bulletin 1998/31**

(51) Int. Cl.$^6$: **C07C 17/42**, C08J 9/06,
C08K 5/02

(21) Numéro de dépôt: 93202682.6

(22) Date de dépôt: 16.09.1993

(54) **Compositions stabilisées comprenant des hydrofluoroalcanes, prémélanges destinés à la préparation de mousses polymériques et mousses polymériques obtenues par leur mise en oeuvre**

Stabilisierte Zusammensetzungen, die Fluorkohlenwasserstoffe enthalten, Vormischungen zur Herstellung von Polymerschäumen und erhaltene Polymerschäume

Stabilized compositions containing hydrofluoroalkanes, premixtures for the preparation of polymeric foams and polymeric foams obtained thereby

(84) Etats contractants désignés:
**AT BE DE DK ES FR GB GR IE IT NL PT**

(30) Priorité: **29.09.1992 BE 9200847**

(43) Date de publication de la demande:
**06.04.1994 Bulletin 1994/14**

(73) Titulaire:
**SOLVAY (Société Anonyme)**
**B-1050 Bruxelles (BE)**

(72) Inventeurs:
• **Barthelemy, Pierre**
**B-1315 Pietrebais (BE)**
• **Leroy, Annie**
**B-6637 Fauvillers (BE)**

(74) Mandataire:
**Jacques, Philippe et al**
**Solvay S.A.**
**Département Propriété Industrielle**
**310, rue de Ransbeek**
**1120 Bruxelles (BE)**

(56) Documents cités:
**WO-A-92/10453**           **US-A- 5 169 996**

## Description

La présente invention concerne des compositions stabilisées comprenant au moins un hydrofluoroalcane.

Les hydrocarbures chlorofluorés entièrement halogénés (CFC) suspectés d'avoir un effet néfaste sur la couche d'ozone peuvent être substitués dans bon nombre d'applications, telles que par exemple l'utilisation comme agent gonflant pour la préparation de mousses, comme fluide caloporteur ou comme propulseur, par des hydrocarbures fluorés comprenant au moins un atome d'hydrogène, encore appelés hydrofluoroalcanes (HFA).

Les HFA peuvent se dégrader dans certaines conditions, de sorte que leur utilisation dans les domaines mentionnés ci-dessus pose encore un problème de stabilité.

Par ailleurs, il est bien connu que les mousses de polyuréthane ou de polyisocyanurate peuvent être préparées en faisant réagir un polyisocyanate avec une quantité appropriée d'un polyol ou d'un mélange de polyols, en présence d'un agent gonflant constitué d'un liquide volatil, lequel est vaporisé par la chaleur libérée par la réaction entre l'isocyanate et le polyol. Il est aussi bien connu que cette réaction est favorisée par l'utilisation de catalyseurs, tels que des amines ou des composés d'étain et que la formation de la mousse peut être améliorée par addition de composés tensioactifs. D'autres additifs peuvent aussi être mis en oeuvre, tels que notamment des agents retardateurs de flamme.

Il est de pratique courante, dans le domaine des mousses de polyuréthane, de préparer des prémélanges de certains composants utilisés ultérieurement pour préparer la mousse. Habituellement, les quantités appropriées de polyol, d'agent gonflant, de catalyseur, de surfactant et d'agent retardateur de flamme sont mélangées pour former un prémélange. Ce prémélange et la quantité adéquate de polyisocyanate sont vendus dans deux réservoirs distincts. L'utilisateur final n'a plus alors qu'à mélanger les contenus des deux réservoirs pour fabriquer la mousse. D'autre part, dans les grosses unités de production de mousses, il est habituel de stocker le mélange contenant le polyol et l'agent gonflant. Ce mélange liquide possède une viscosité plus faible que celle du polyol pur et est dès lors plus facile à pomper et à doser vers l'aire de fabrication de la mousse. Quelle que soit la pratique utilisée, des précautions particulières doivent être prises. On a en effet constaté que, dans les prémélanges comprenant des polyols, l'hydrofluoroalcane utilisé comme agent gonflant est susceptible d'être dégradé en divers produits de dégradation gênants. Le 1,1-dichloro-1-fluoroéthane, par exemple, se dégrade en présence de polyols, notamment en 1-chloro-1-fluoroéthane, en chlorure de vinylidène et en chlorofluorure de vinylidène. Des hydracides peuvent également être générés par cette dégradation.

Un problème similaire de stabilité existe pour les compositions solvantes obtenues par mélange d'au moins un HFA avec divers composés organiques, notamment des alcools.

La demande internationale WO 92/10453 mentionne différents stabilisants du 1,1-dichloro-1-fluoroéthane.

La présente invention a pour objet de procurer des compositions d'hydrofluoroalcanes de stabilité améliorée, plus particulièrement à l'encontre de la dégradation induite par les composés hydroxylés.

L'invention concerne dès lors des compositions comprenant au moins un hydrofluoroalcane et au moins un stabilisant de l'hydrofluoroalcane, caractérisées en ce que le stabilisant est choisi parmi les quinones, les composés comportant un groupement nitroso et leurs mélanges.

La demande de brevet européen EP-A-540840, publiée le 12 mai 1993, décrit des compositions comprenant du 1,1-dichloro-1-fluoroéthane et de la 1,4-benzoquinone ou de la 2,6-di-t-butylbenzoquinone.

Par hydrofluoroalcane, on entend généralement désigner tout hydrocarbure halogéné saturé, de type acyclique ou alicyclique, comprenant au moins un atome d'hydrogène et au moins un atome de fluor. Ces hydrofluoroalcanes peuvent ou non comprendre en outre un ou plusieurs atomes de chlore ou de brome. De préférence, ils ne comprennent pas de brome. De manière particulièrement préférée, ils comprennent au moins un atome de chlore.

Des hydrofluoroalcanes utilisables dans les compositions selon i'invention sont ceux qui comprennent de 1 à 6 atomes de carbone et qui répondent à la formule générale $C_aH_bF_cX_d$ dans laquelle X est Cl et/ou Br, de préférence Cl, a est un nombre entier de 1 à 6, b est un nombre entier de 1 à 13, c est un nombre entier de 1 à 13 et d est un nombre entier de 0 à 8, avec $b + c + d = 2a + 2$ lorsque l'hydrofluoroalcane est acyclique et avec $b + c + d = 2a$ lorsque l'hydrofluoroalcane est alicyclique. Les compositions selon l'invention comprennent particulièrement des hydrofluoroalcanes acycliques répondant à la formule générale ci-dessus dans laquelle X est Cl, a est un nombre entier de 1 à 4, b est un nombre entier de 1 à 9, c est un nombre entier de 1 à 9 et d est un nombre entier de 0 à 5. Elles comprennent plus particulièrement des hydrofluoroalcanes acycliques répondant à la formule générale ci-dessus dans laquelle X est Cl, a est un nombre entier égal à 2 ou 3, b est un nombre entier de 1 à 6, c est un nombre entier de 1 à 6 et d est un nombre entier de 1 à 4. A titre d'exemples, l'hydrofluoroalcane des compositions selon l'invention peut être sélectionné parmi les composés de formule brute $CHClF_2$, $CH_2F_2$, $CH_3CCl_2F$, $CH_3CClF_2$, $CH_3CHF_2$, $CH_3CF_3$, $CH_2FCH_2F$, $CH_2FCHF_2$, $CH_2FCF_3$, $CHF_2CCl_3$, $CHF_2CF_3$, $CHCl_2CF_3$, $CHF_2CHF_2$, $CF_3CHClF$, $CF_3CF_2CHCl_2$, $CF_2ClCF_2CHClF$, $CF_3CH_2CF_2CH_3$ et $CF_3CH_2CH_2CF_3$. Le 1,1-dichloro-1-fluoroéthane (HFA-141b) est préféré. D'excellents résultats sont obtenus, notamment lorsque les compositions selon l'invention sont mises en oeuvre comme agent gonflant dans des prémélanges à base de polyols, destinés à la fabrication de mousses de polyuréthane, tout particulièrement lorsque l'hydrofluoroalcane est le 1,1-dichloro-1-fluoroéthane.

Les compositions stabilisées selon l'invention peuvent comprendre un seul hydrofluoroalcane ou un mélange

2

d'hydrofluoroalcanes différents.

Les quinones utilisables dans les compositions selon l'invention peuvent être non substituées ou substituées par des groupes alkyles en $C_1$-$C_{10}$. Lorsque les quinones sont substituées par des groupes alkyles, ceux-ci sont de préférence ramifiés. Des exemples de quinones utilisables pour stabiliser les compositions selon l'invention comprennent notamment la 1,4-benzoquinone, la tétrahydro-1,4-benzoquinone, l'anthraquinone, la tétrahydroanthraquinone, l'octahydroanthraquinone, éventuellement substituées par un ou plusieurs groupements alkyles en $C_1$-$C_{10}$. On met en particulier en oeuvre la 1,4-benzoquinone, la 2,6-diméthylbenzoquinone, la 2-sec-amylanthraquinone, la 2-tert-amylanthraquinone, la 2-tert-amyltétrahydroanthraquinone ou la 2-tert-amyloctahydroanthraquinone. On met tout particulièrement en oeuvre la 1,4-benzoquinone, la 2-tert-amyltétrahydroanthraquinone ou la 2-tert-amyloctahydroanthraquinone. D'excellents résultats ont été obtenus avec la 1,4-benzoquinone.

Les composés comportant un groupement nitroso utilisables pour stabiliser les compositions selon l'invention sont des composés acycliques, alicycliques, aromatiques ou hétérocycliques comportant un groupement nitroso (-N=O) et éventuellement substitués par d'autres groupements, tels que des groupements alkyle, amino ou hydroxy. A titre d'exemples, on peut mentionner le 2-méthyl-2-nitrosopropane, le nitrosobenzène, le 2-nitrosotoluène, le 3-nitrosodurène, le 4-nitrosophénol, le 1-nitroso-2-naphtol, le 2-nitroso-1-naphtol, la N-nitrosodiméthylamine, la N-nitrosodiéthylamine, la 4-nitrosodiphénylamine, la N-nitrosodiéthanolamine, la 4-nitroso-N,N-diméthylaniline, la 4-nitroso-N,N-diéthylaniline, la 1-nitrosopyrrolidine, la N-nitrosomorpholine, la 5-nitroso-2,4,6-triaminopyrimidine. On choisit plus particulièrement le 2-méthyl-2-nitrosopropane, le 2-nitroso-1-naphtol ou la N-nitrosodiéthylamine. D'excellents résultats ont été obtenus avec le 2-méthyl-2-nitrosopropane.

Dans les compositions selon l'invention, le stabilisant doit être mis en oeuvre en quantité appropriée pour stabiliser l'hydrofluoroalcane. La quantité optimum de stabilisant à mettre en oeuvre dépend de divers paramètres, parmi lesquels figurent notamment l'hydrofluoroalcane et le stabilisant sélectionnés. Elle peut être déterminée aisément dans chaque cas particulier. En pratique, on utilise généralement au moins 0,01 % en poids de chaque stabilisant par rapport au poids total d'hydrofluoroalcane compris dans la composition. De préférence, on en utilise au moins 0,05 %. De manière particulièrement préférée, on en utilise au moins 0,1 %. Par ailleurs, on ne dépasse habituellement pas 10 % en poids de chaque stabilisant par rapport au poids total d'hydrofluoroalcane compris dans la composition. De préférence, on ne dépasse pas 5 %. De manière particulièrement préférée, on ne dépasse pas 2 %.

Les compositions selon l'invention peuvent contenir plusieurs stabilisants sélectionnés parmi les quinones et les composés comportant un groupement nitroso. Les compositions selon l'invention peuvent être constituées essentiellement de l'hydrofluoroalcane et du stabilisant. En variante, d'autres agents stabilisants et/ou d'autres additifs peuvent également être ajoutés aux compositions stabilisées selon l'invention. La nature des additifs mis en oeuvre dépend principalement de l'utilisation à laquelle sont destinées les compositions selon l'invention.

Les compositions selon l'invention peuvent avantageusement être mises en oeuvre comme agent gonflant pour la préparation de mousses polymériques. Elles peuvent aussi être mises en oeuvre comme agent de nettoyage, par exemple comme solvant de dégraissage de pièces métalliques. Dans ce cas, les additifs sont, par exemple, des alcools inférieurs comme le méthanol, l'éthanol, le propanol ou l'isopropanol. Elles peuvent également être utilisées comme fluide caloporteur dans les appareils de réfrigération ou comme gaz propulseur pour produits aérosols.

Les compositions stabilisées selon l'invention peuvent en particulier être mises en oeuvre comme agent gonflant pour la préparation de mousses de polyuréthane ou de polyisocyanurate.

L'invention concerne également des prémélanges destinés à la préparation de mousses polymériques, telles que des mousses de polyuréthane ou de polyisocyanurate, comprenant comme agent gonflant, une composition comprenant au moins un hydrofluoroalcane et au moins un stabilisant de l'hydrofluoroalcane choisi parmi les quinones et les composés comportant un groupement nitroso.

L'invention concerne particulièrement des prémélanges qui comprennent au moins un polyol et qui sont destinés à la fabrication de mousses de polyuréthane. Une large gamme de polyols déjà divulgués dans l'art antérieur peuvent être mis en oeuvre dans ces prémélanges, tels que des polyéthers polyols et des polyesters polyols. La proportion d'agent gonflant par rapport au polyol dans les prémélanges va varier, notamment selon l'application, le type de mousse préparé, la nature du polyol, la nature de l'hydrofluoroalcane compris dans la composition mise en oeuvre comme agent gonflant et encore selon d'autres paramètres. Elle peut être déterminée facilement dans chaque cas particulier. En pratique, on utilise généralement de 1 à 50 parts en poids de la composition comprenant l'hydrofluoroalcane et le stabilisant de l'hydrofluoroalcane pour 100 parts en poids de polyol. D'excellents résultats ont été obtenus avec des prémélanges comprenant un polyol et du 1,1-dichloro-1-fluoroéthane stabilisé selon l'invention, destinés à la fabrication de mousses de polyuréthane rigides. Ils se sont avérés particulièrement stables, ne donnant lieu en particulier qu'à une formation très réduite de 1-chloro-1-fluoroéthane (HFA-151a). De tels prémélanges peuvent éventuellement comprendre un ou plusieurs autres agents gonflants, en plus de l'hydrofluoroalcane stabilisé selon l'invention. Ces prémélanges peuvent éventuellement n'être constitués que des quantités appropriées du polyol et de la composition stabilisée selon l'invention. Cependant, en règle générale, ils comprennent, outre des quantités appropriées du polyol et de la composition stabilisée selon l'invention, des quantités nécessaires de surfactants, de catalyseurs, d'agents retardateurs de flamme et éventuellement d'autres additifs utilisés habituellement pour préparer des mousses de polyuréthane par réaction avec des polyisocyanates.

L'invention concerne également des mousses de polyuréthane ou de polyisocyanurate obtenues par mise en oeuvre d'une composition ou d'un prémélange conformes à l'invention, tels que définis plus haut.

Les exemples 2 à 9 qui suivent sont donnés pour illustrer l'invention, de manière non limitative. L'exemple 1(C) est donné à titre de référence, en absence de stabilisant.

Exemple 1(C)

Un prémélange pour la préparation de mousses de polyuréthane a été préparé selon la composition pondérale suivante :

- 50 parties de polyol aminé ARCOL 3770 de ARCO
- 50 parties de polyol aminé VORANOL RA 640 de DOW
- 1 partie d'eau
- 2 parties de surfactant siliconé B 1048 de GOLDSCHMIDT
- 2 parties de N-méthylmorpholine
- 1,5 partie de N,N-diméthylcyclohexylamine
- 24 parties de 1,1-dichloro-1-fluoroéthane.

Une quantité prédéterminée de ce mélange a été enfermée dans un flacon en verre maintenu à une température constante de 50 °C durant 12 jours.

Un échantillon a alors été prélevé et son analyse par chromatographie en phase gazeuse a révélé la présence de 294 mg de 1-chloro-1-fluoroéthane par kg de 1,1-dichloro-1-fluoroéthane.

Exemples 2 à 9

Dans un prémélange identique à celui de l'exemple 1(C), on a ajouté avant vieillissement des quantités variables de différents stabilisants de manière à obtenir des prémélanges selon l'invention.

Après vieillissement dans des conditions identiques à celles de l'exemple 1(C), on a mesuré par chromatographie en phase gazeuse la teneur en 1-chloro-1-fluoroéthane. Les résultats sont présentés dans le tableau ci-après.

Par comparaison avec l'exemple 1(C), les exemples 2 à 9 selon l'invention illustrent que la quantité de 1-chloro-1-fluoroéthane formé est très nettement inférieure et donc que la stabilité du 1,1-dichloro-1-fluoroéthane est très sensiblement meilleure dans les prémélanges selon l'invention.

| Exemple | Composé stabilisant | | Teneur en HFA-151a (mg/kg HFA-141b) |
|---|---|---|---|
| | Nature | Quantité (% poids de HFA-141b) | |
| 1(C) | - | - | 294 |
| 2 | 1,4-benzoquinone | 5 % | < 5 |
| 3 | 1,4-benzoquinone | 0,1 % | < 5 |
| 4 | 1,4-benzoquinone | 0,05 % | 11 |
| 5 | 2-méthyl-2-nitrosopropane | 5 % | < 5 |
| 6 | 2-méthyl-2-nitrosopropane | 0,1 % | < 5 |
| 7 | 2-méthyl-2-nitrosopropane | 0,05 % | 7 |
| 8 | 2-amyltétrahydroanthraquinone (*) | 5 % | < 5 |
| 9 | 2-tert-amyloctahydroanthraquinone | 5 % | < 5 |

(*) : mélange des isoméres tert-amyl (60 %) et sec-amyl (40 %)

**Revendications**

**Revendications pour les Etats contractants suivants : DE, DK, ES, GR, IE, PT**

1.  Compositions comprenant au moins un hydrofluoroalcane et au moins un stabilisant de l'hydrofluoroalcane, caractérisées en ce que le stabilisant est choisi parmi les quinones, les composés comportant un groupement nitroso et leurs mélanges.

2.  Compositions selon la revendication 1 essentiellement constituées de l'hydrofluoroalcane et du stabilisant.

3.  Compositions selon la revendication 1 ou 2 comprenant du 1,1-dichloro-1-fluoroéthane.

4.  Compositions selon l'une quelconque des revendications 1 à 3, caractérisées en ce que la quinone est choisie parmi la 1,4-benzoquinone, la 2,6-diméthylbenzoquinone, la 2-sec-amylanthraquinone, la 2-tert-amylanthraquinone, la 2-tert-amyltétrahydroanthraquinone et la 2-tert-amyloctahydroanthraquinone.

5.  Compositions selon l'une quelconque des revendications 1 à 3, caractérisées en ce que le composé comportant un groupement nitroso est choisi parmi le 2-méthyl-2-nitrosopropane, le 2-nitroso-1-naphtol et la N-nitrosodiéthylamine.

6.  Compositions selon l'une quelconque des revendications 1 à 5, caractérisées en ce qu'elles contiennent de 0,01 à 10 % en poids de chaque stabilisant par rapport au poids total de l'hydrofluoroalcane.

7.  Utilisation des compositions selon l'une quelconque des revendications 1 à 6 comme agent gonflant pour la préparation de mousses de polyuréthane ou de polyisocyanurate.

8.  Prémélanges destinés à la préparation de mousses de polyuréthane ou de polyisocyanurate comprenant, comme agent gonflant, une composition conforme à l'une quelconque des revendications 1 à 6.

9.  Prémélanges selon la revendication 8 comprenant au moins un polyol et destinés à la fabrication de mousses de polyuréthane.

10. Prémélanges selon la revendication 8 ou 9 dans lesquels l'agent gonflant comprend du 1,1-dichloro-1-fluoroéthane.

11. Mousses de polyuréthane ou de polyisocyanurate obtenues par mise en oeuvre de toute composition selon l'une quelconque des revendications 1 à 6 ou de tout prémélange selon l'une quelconque des revendications 8 à 10.

**Revendications pour les Etats contractants suivants : AT, BE, FR, GB, IT, NL**

1.  Compositions comprenant au moins un hydrofluoroalcane et au moins un stabilisant de l'hydrofluoroalcane, caractérisées en ce que le stabilisant est choisi parmi les quinones, les composés comportant un groupement nitroso et leurs mélanges, à l'exception des compositions comprenant un hydrochlorofluoroalcane et un stabilisant choisi parmi la 1,4-benzoquinone et la 2,6-di-t-butylbenzoquinone.

2.  Compositions selon la revendication 1 essentiellement constituées de l'hydrofluoroalcane et du stabilisant.

3.  Compositions selon la revendication 1 ou 2 comprenant du 1,1-dichloro-1-fluoroéthane.

4.  Compositions selon l'une quelconque des revendications 1 à 3, caractérisées en ce que la quinone est choisie parmi la 1,4-benzoquinone, la 2,6-diméthylbenzoquinone, la 2-sec-amylanthraquinone, la 2-tert-amylanthraquinone, la 2-tert-amyltétrahydroanthraquinone et la 2-tert-amyloctahydroanthraquinone.

5.  Compositions selon l'une quelconque des revendications 1 à 3, caractérisées en ce que le composé comportant un groupement nitroso est choisi parmi le 2-méthyl-2-nitrosopropane, le 2-nitroso-1-naphtol et la N-nitrosodiéthylamine.

6.  Compositions selon l'une quelconque des revendications 1 à 5, caractérisées en ce qu'elles contiennent de 0,01 à 10 % en poids de chaque stabilisant par rapport au poids total de l'hydrofluoroalcane.

7. Utilisation des compositions selon l'une quelconque des revendications 1 à 6 comme agent gonflant pour la préparation de mousses de polyuréthane ou de polyisocyanurate.

8. Prémélanges destinés à la préparation de mousses de polyuréthane ou de polyisocyanurate comprenant, comme agent gonflant, une composition conforme à l'une quelconque des revendications 1 à 6.

9. Prémélanges selon la revendication 8 comprenant au moins un polyol et destinés à la fabrication de mousses de polyuréthane.

10. Prémélanges selon la revendication 8 ou 9 dans lesquels l'agent gonflant comprend du 1,1-dichloro-1-fluoroéthane.

11. Mousses de polyuréthane ou de polyisocyanurate obtenues par mise en oeuvre de toute composition selon l'une quelconque des revendications 1 à 6 ou de tout prémélange selon l'une quelconque des revendications 8 à 10.

**Claims**

**Claims for the following Contracting States : DE, DK, ES, GR, IE, PT**

1. Compositions comprising at least one hydrofluoroalkane and at least one stabilizing agent of the hydrofluoroalkane, characterized in that the stabilizing agent is chosen from quinones, compounds containing a nitroso group and their mixtures.

2. Compositions according to Claim 1, consisting essentially of the hydrofluoroalkane and the stabilizing agent.

3. Compositions according to Claim 1 or 2, comprising 1,1-dichloro-1-fluoroethane.

4. Compositions according to any one of Claims 1 to 3, characterized in that the quinone is chosen from 1,4 benzoquinone, 2,6-dimethylbenzoquinone, 2-sec-amylanthraquinone, 2-tert-amylanthraquinone, 2-tert-amyltetrahydroanthraquinone and 2-tert-amyloctahydroanthraquinone.

5. Compositions according to any one of Claims 1 to 3, characterized in that the compound containing a nitroso group is chosen from 2-methyl-2-nitrosopropane, 2 nitroso-1-naphthol and N-nitrosodiethylamine.

6. Compositions according to any one of Claims 1 to 5, characterized in that they contain from 0.01 to 10 % by weight of each stabilizing agent with respect to the total weight of the hydrofluoroalkane.

7. Use of the compositions according to any one of Claims 1 to 6 as blowing agents for the preparation of polyurethane or polyisocyanurate foams.

8. Premixtures intended for the preparation of polyurethane or polyisocyanurate foams comprising, as blowing agent, a composition in accordance with any one of Claims 1 to 6.

9. Premixtures according to Claim 8 comprising at least one polyol and intended for the manufacture of polyurethane foams.

10. Premixtures according to Claim 8 or 9, in which the blowing agent comprises 1,1-dichloro-1-fluoroethane.

11. Polyurethane or polyisocyanurate foams obtained by use of any composition according to any one of Claims 1 to 6 or of any premixture according to any one of Claims 8 to 10.

**Claims for the following Contracting States : AT, BE, FR, GB, IT, NL**

1. Compositions comprising at least one hydrofluoroalkane and at least one stabilizing agent of the hydrofluoroalkane, characterized in that the stabilizing agent is chosen from quinones, compounds containing a nitroso group and their mixtures, the compositions comprising one hydrochlorofluoroalkane and a stabilizing agent chosen from 1,4 benzoquinone and 2,6-dimethylbenzoquinone being excepted.

2. Compositions according to Claim 1, consisting essentially of the hydrofluoroalkane and the stabilizing agent.

3. Compositions according to Claim 1 or 2, comprising 1,1-dichloro-1-fluoroethane.

4. Compositions according to any one of Claims 1 to 3, characterized in that the quinone is chosen from 1,4 benzoquinone, 2,6-dimethylbenzoquinone, 2-sec-amylanthraquinone, 2-tert-amylanthraquinone, 2-tert-amyltetrahydroanthraquinone and 2-tert-amyloctahydroanthraquinone

5. Compositions according to any one of Claims 1 to 3, characterized in that the compound containing a nitroso group is chosen from 2-methyl-2-nitrosopropane, 2 nitroso-1-naphthol and N-nitrosodiethylamine.

6. Compositions according to any one of Claims 1 to 5, characterized in that they contain from 0.01 to 10 % by weight of each stabilizing agent with respect to the total weight of the hydrofluoroalkane.

7. Use of the compositions according to any one of Claims 1 to 6 as blowing agents for the preparation of polyurethane or polyisocyanurate foams.

8. Premixtures intended for the preparation of polyurethane or polyisocyanurate foams comprising, as blowing agent, a composition in accordance with any one of Claims 1 to 6.

9. Premixtures according to Claim 8 comprising at least one polyol and intended for the manufacture of polyurethane foams.

10. Premixtures according to Claim 8 or 9, in which the blowing agent comprises 1,1-dichloro-1-fluoroethane.

11. Polyurethane or polyisocyanurate foams obtained by use of any composition according to any one of Claims 1 to 6 or of any premixture according to any one of Claims 8 to 10.

**Patentansprüche**

**Patentansprüche für folgende Vertragsstaaten : DE, DK, ES, GR, IE, PT**

1. Zusammensetzungen, die Wenigstens einen Fluorkohlenwasserstoff und wenigstens einen Stabilisator für den Fluorkohlenwasserstoff umfassen, dadurch gekennzeichnet, daß der Stabilisator unter den Chinonen, den Verbindungen, die eine Nitrosogruppe umfassen$_1$ und deren Gemischen ausgewählt ist.

2. Zusammensetzungen gemäß Anspruch 1, die im wesentlichen aus dem Fluorkohlenwasserstoff und dem Stabilisator bestehen.

3. Zusammensetzungen gemäß Anspruch 1 oder 2, die 1,1-Dichlor-1-fluorethan umfassen.

4. Zusammensetzungen gemäß einem der Ansprüche 1 bis 3, dadurch gekennzeichnet, daß das Chinon unter 1,4-Benzochinon, 2,6-Dimethylbenzochinon, 2-sec-Amylanthrachinon, 2-tert-Amylanthrachinon, 2-tert-Amyltetrahydroanthrachinon und 2-tert-Amyloctahydroanthrachinon ausgewählt ist.

5. Zusammensetzungen gemäß einem der Ansprüche 1 bis 3, dadurch gekennzeichnet, daß die Verbindung, die eine Nitrosogruppe umfaßt, unter 2-Methyl-2-nitrosopropan, 2-Nitroso-1-naphthol und N-Nitrosodiethylamin ausgewählt ist.

6. Zusammensetzungen gemäß einem der Ansprüche 1 bis 5, dadurch gekennzeichnet, daß sie, bezogen auf das Gesamtgewicht des Fluorkohlenwasserstoffs, 0,01 bis 10 Gew.-% von jedem Stabilisator enthalten.

7. Verwendung der Zusammensetzungen gemäß einem der Ansprüche 1 bis 6 als Treibmittel für die Herstellung von Polyurethan- oder Polyisocyanuratschäumen.

8. Vormischungen, die zur Herstellung von Polyurethan- oder Polyisocyanuratschäumen bestimmt sind, die als Treibmittel eine Zusammensetzung gemäß einem der Ansprüche 1 bis 6 umfassen.

9. Vormischungen gemäß Anspruch 8, die wenigstens ein Polyol umfassen und zur Herstellung von Polyurethanschäumen bestimmt sind.

10. Vormischungen gemäß Anspruch 8 oder 9, in denen das Treibmittel 1,1-Dichlor-1-fluorethan umfaßt.

11. Polyurethan- oder Polyisocyanuratschäume, die durch Verwendung jeder Zusammensetzung gemäß einem der Ansprüche 1 bis 6 oder jeder Vormischung gemäß einem der Ansprüche 8 bis 10 erhalten werden.

**Patentansprüche für folgende Vertragsstaaten : AT, BE, FR, GB, IT, NL**

1. Zusammensetzungen, die wenigstens einen Fluorkohlenwasserstoff und wenigstens einen Stabilisator für den Fluorkohlenwasserstoff umfassen, dadurch gekennzeichnet, daß der Stabilisator unter den Chinonen, den Verbindungen, die eine Nitrosogruppe umfassen, und deren Gemischen ausgewählt ist, mit Ausnahme der Zusammensetzungen, die einen Chlorfluorkohlenwasserstoff und einen Stabilisator umfassen, der unter 1,4-Benzochinon und 2,6-Di-tert-butylbenzochinon ausgewählt ist.

2. Zusammensetzungen gemäß Anspruch 1, die im wesentlichen aus dem Fluorkohlenwasserstoff und dem Stabilisator bestehen.

3. Zusammensetzungen gemäß Anspruch 1 oder 2, die 1,1-Dichlor-1-fluorethan umfassen.

4. Zusammensetzungen gemäß einem der Ansprüche 1 bis 3, dadurch gekennzeichnet, daß das Chinon unter 1,4-Benzochinon, 2,6-Dimethylbenzochinon, 2-sec-Amylanthrachinon, 2-tert-Amylanthrachinon, 2-tert-Amyltetrahydroanthrachinon und 2-tert-Amyloctahydroanthrachinon ausgewählt ist.

5. Zusammensetzungen gemäß einem der Ansprüche 1 bis 3, dadurch gekennzeichnet, daß die Verbindung, die eine Nitrosogruppe umfaßt, unter 2-Methyl-2-nitrosopropan, 2-Nitroso-1-naphthol und N-Nitrosodiethylamin ausgewählt ist.

6. Zusammensetzungen gemäß einem der Ansprüche 1 bis 5, dadurch gekennzeichnet, daß sie, bezogen auf das Gesamtgewicht des Fluorkohlenwasserstoffs, 0,01 bis 10 Gew.-% von jedem Stabilisator enthalten.

7. Verwendung der Zusammensetzungen gemäß einem der Ansprüche 1 bis 6 als Treibmittel für die Herstellung von Polyurethan- oder Polyisocyanuratschäumen.

8. Vormischungen, die zur Herstellung von Polyurethan- oder Polyisocyanuratschäumen bestimmt sind, die als Treibmittel eine Zusammensetzung gemäß einem der Ansprüche 1 bis 6 umfassen.

9. Vormischungen gemäß Anspruch 8, die wenigstens ein Polyol umfassen und zur Herstellung von Polyurethanschäumen bestimmt sind.

10. Vormischungen gemäß Anspruch 8 oder 9, in denen das Treibmittel 1,1-Dichlor-1-fluorethan umfaßt.

11. Polyurethan- oder Polyisocyanuratschäume, die durch Verwendung jeder Zusammensetzung gemäß einem der Ansprüche 1 bis 6 oder jeder Vormischung gemäß einem der Ansprüche 8 bis 10 erhalten werden.